# EUROPEAN PATENT APPLICATION

(11) **EP 0 879 894 A2**
(43) Date of publication of application: **25.11.1998**
(21) Application number: 98108500.4
(22) Date of filing: 11.05.1998
(51) Int. Cl.: C12Q 1/68, C12Q 1/70

(54) **Sample preparation for nucleic acid based diagnostic tests**

(30) Priority: 20.05.1997 EP 97108118
(71) Applicant: F. HOFFMANN-LA ROCHE AG, 4070 Basel (CH)
(72) Inventor: Perrin, Luc Henri, 1226 Thônex (CH)
(74) Representative: Wächter, Dieter Ernst, Dr.

(57) **Abstract**

The present invention provides a method for increasing the sensitivity of a diagnostic test for the detection of a nucleic acid of an infectious agent in pooled samples of liquid biological material, characterized in that the nucleic acid is selectively precipitated by adding a precipitating agent, whereby the preferred precipitating agent is polyethylene glycol in a molecular weight range of 1'000 to 10'000 at a final concentration of 1 to 5 % (w/v). The precipitation efficiency may be increased by the addition of mono- or divalent salts. Preferred examples of such salts are NaCl or KCl, preferably these salts are added so that the final salt concentration is 200 to 450 mM, most preferably 270 to 325 mM salt. The invention also relates also to the use of the said method in a diagnostic test as well as to kits which are useful for practicing the said method.

## Description

The present invention provides a novel sample preparation method for pooled samples of liquid biological material, which method leads to an increase in the sensitivity of nucleic acid based diagnostic test. The invention also provides kits which are particularly suitable for performing said method.

In order to increase the safety of blood bank products official authorities of various countries have requested that such products be analyzed by nucleic acid based diagnostic tests before they are administered to patients. This in view of the fact that the antibodies raised against an infectious virus can often not be detected for several weeks or months after the actual infection has taken place. On the other hand nucleic acid based diagnostic tests, which often involve the amplification of the nucleic acid of the infectious agents, e.g. by the polymerase chain reaction (PCR), are capable to detect the presence of the infectious agent very early after infection.

Thus, the detection of human immunodeficiency virus (HIV) and hepatitis C virus (HCV) relies on screening for antibodies by enzyme immunoassay (EIA). While the sensitivities of HIV and HCV antibody detection assays have improved over recent years, a window period persists between infection and detectable seroconversion. Although roughly one third of HCV seroconversions take place as early as 2 weeks after infection (Lim et al., J. Haematol., 1991, 78:398), anti-HCV antibodies may develop as late as 6 months after infection (Van der Poel et al., Vox Sang, 1992, 62:208 and Farci et al., N. Eng. J. Med., 1991, 325:98). The contemporary anti-HIV-1 antibody assays become positive on average 22 days after infectiousness (Busch et al., Transfusion, 1995, 35:91). Thus, anti-HCV and anti-HIV antibodies negative donated blood collected during the infectious window period, may escape the blood safety screening procedures. Reduction of the window period can be achieved by assays targeting viral nucleic acids. In particular, it has been evaluated that PCR assays reduce the window period by 11 days for HIV-1 infection (Busch et al., Transfusion, 1995, 35:91) and 59 days for HCV infection (Alter HJ, Blood, 1995, 85:1681).

The risk of transmitting HCV and HIV from antibody screened blood has been estimated as 1 in 493 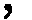 000 for HIV-1 and 1 in 103 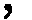 000 for HCV (Schreiber et al., N. Eng. J. Med., 1996, 334:1685). This low incidence has raised concern on the cost-effectiveness of a systematic screening of blood by PCR assays (AuBuchon et al., Transfusion, 1997, 37:45). Thus, there is a need for a simplified and low cost nucleic acid amplification-based screening assay.

Unfortunately nucleic acid amplification based methods still involve several steps which are not easy to automate in such a way that a machine can handle a large number of test samples at the same time. It has therefore been proposed to pool samples and to perform the nucleic acid based diagnostic test with such pooled samples. Pooled samples which are found positive are then further analyzed, e.g. in that each sample of the pool is tested individually, or, in that pools of different overlapping samples are analyzed in order to find out which of the sample in the pool is positive. Thus, e.g. a positive sample C can easily be determined as being positive by analyzing pools of samples AB, AC and BC, because in such a pooling test the pools AC and BC should be found to be positive. Of course in the latter pooling procedure safety can be increased by making a confirmatory test where sample C is tested individually.

The major disadvantage of the pooling methods is the decreased sensitivity which occurs upon pooling.

A need to increase the sensitivity of nucleic acid based diagnostic tests has also been encountered in the monitoring of the treatment of infected individuals. In order to assess the optimal time for changing or stopping drug treatment it is necessary to determine at what time the virus nucleic acid has fallen under the detectable level. Schockmel et al. have addressed this problem by using a boosted assay format for the detection of HIV-1 RNA and were able to increase the sensitivity of detection to 20 copies of RNA per milliliter of plasma (J. of Acquired Immune Deficiency Syndrome and Human Retrovirology, 1997, 14:149-183).

The problem of further increasing the sensitivity of nucleic acid based diagnostic tests is solved by the present invention, the basis of which invention is the methods as defined in the appended set of claims. More particularly the present invention provides a method for increasing the sensitivity of a diagnostic test for the detection of a nucleic acid of an infectious agent in pooled samples of liquid biological material, whereby this method is characterized in that the nucleic acid is selectively precipitated by adding a precipitating agent (e.g. polyethylene glycol, dextran sulfate or ammonium sulfate). The preferred precipitating agent is polyethylene glycol (PEG) in a molecular weight range of 1'000 to 10'000 at a final concentration of 1 to 5 % (w/v). More preferably the method is characterized in that the precipitating agent is polyethylene glycol in a molecular weight range of 7'000 to 9'000 at a final concentration of 2.0 to 4.0 % (w/v), even more preferably at a final concentration of 2.5 to 3.0 % (w/v), most preferably PEG 6000 at a final concentration of about 2.8 to 2.9 % (w/v) in the pooled samples. In the method of the present invention a mono- or divalent salt, preferably a monovalent salt may be added to enhance the precipitation of the nucleic acid. Preferred monovalent salt are NaCl and KCl. The amount of salt to be added depends from the salt concentration already present in the biological samples, which is in most cases the physiological salt concentration of about 145 mM salt. The final salt concentration in the mixture is preferably 200 to 450 mM, most preferably 270 to 325 mM.

In the method of the present invention the precipitation is preferably effected at a temperature below 5°C, e.g. at 0° to 4°C.

The method of the present invention is suitable to effect the simultaneous precipitation of multiple types of virus, such as HCV and HIV (e.g. co-infection samples). For control purposes also a defined virus, prepared e.g. by means of the recombinant DNA technology may be used as an internal control or for quantitation purposes.

The precipitation efficiency may be further enhanced by the addition of carrier nucleic acid. Examples of such carrier nucleic acid are calf thymus DNA or salmon sperm DNA. Said carrier nucleic acid may also be of a defined sequence which allows e.g. to control the effectiveness of the precipitation of the nucleic acid of the infectious agent. Thus, for example a fixed amount of phage λ DNA may be added for that purpose. In order to control the effectiveness of the whole diagnostic assay procedure including the method of the present invention, the amplification step and the detection step, a separate nucleic acid can be added as an internal control. Said separate nucleic acid may be an RNA, a DNA or a synthetic oligonucleotide. In case the nucleic acid of the infectious agent is an RNA the internal control nucleic acid is preferably also an RNA molecule. The internal control nucleic acid has preferably essentially the same length and base composition as the target sequence to be amplified in the nucleic acid of the infectious agent and should comprise at the appropriate positions in the flanking regions sequences which are complementary to the sequence of the primers used for amplifying the target sequence. In this way the target sequence of the nucleic acid of the infectious agent, as well as the internal control nucleic acid sequence, are amplified with the same primers and yield amplification products (amplicons) of the same size. The amplified material can then be tested for the presence of the amplified internal control nucleic acid by using a probe which is specific for the central region of the internal control nucleic acid. In this way the efficiency of the precipitation and/or amplification reactions can be monitored even in samples which do not contain any nucleic acid from the infectious agent. This makes sure that negative samples are indeed negative, i.e. are not caused by faulty precipitation and/or an incomplete amplification reaction. In other words, when the detection step shows the presence of an appropriate amount of the internal control nucleic acid in the sample at the end of the procedure, then it is certain that all steps in the procedure, viz. the precipitation, the amplification and the detection worked properly.

The precipitate obtained by adding the precipitating agent and, if necessary the salt, as indicated above, is then separated from the liquid, whereby preferably a centrifugation step is used.

The next step consists in the lysis of the infectious agents which can be achieved by a number of procedures known to the person skilled in the art. Preferred procedures are the addition of chaotropic agents (e.g. guanidine thiocyanate), detergents (e.g. NP40 or Triton X100), heat treatment or the digestion with a protease such as Proteinase K (preferably in presence of a detergent). In order to stabilize the RNA molecules that are released in the lysis step, inhibitors of RNA degradation (e.g. RNasin®, available from Promega, Madison WI, U.S.A.) may be added to the lysis buffer. The lysis is performed at a temperature of about 15 to 45 °C, preferably at about 37 °C. In the preferred procedure the precipitate is resuspended in 0.5 to 5 ml, preferably about 2.5 ml of lysis buffer. The preferred lysis buffer comprises a chaotropic agent such as guanidine thiocyanate in a suitable buffer and preferably additionally comprises a dithiothreitol or a similar agent. The most preferred lysis buffer is 60 mM Tris/HCl pH 7.5, 68 % w/v guanidine thiocyanate, 3 % w/v dithiothreitol.

The lysis step is followed by an additional purification step which leads to a further removal of PEG (may inhibit PCR) and proteins from the nucleic acids. The purification can be achieved by a number of established procedures, the procedure selected here is based on the use of the standard procedure established for the AMPLICOR™ tests (a line of test systems based on PCR manufactured by F. Hoffmann-La Roche Ltd, Basle, Switzerland). The test is based on the method of Chomczinsky P. and Sacchi N. as described in Anal. Biochem., 1987, 162:156-159. The procedure has however been modified in order to increase the purity of the final nucleic acid product used for the amplification. Thus, e.g. the volume of the lysis reagent has been increased to 2.5 ml.

After the lysis the nucleic acid is precipitated. This is usually done by adding an equal amount of isopropanol (any other C₁ to C₆ alcohol such as e.g. methanol, ethanol, propanol as well as liquid forms [at room temperature] of butanoles, pentanoles and hexanoles may also be used). After a few minutes, preferably after about two minutes the nucleic acid is pelleted by centrifugation. The nucleic acid is then preferably reprecipitated with a solution of about 70% w/v ethanol in water (instead of ethanol any other C₁ to C₆ alcohol such as e.g. methanol, propanol and isopropanol as well as liquid forms [at room temperature] of butanoles, pentanoles and hexanoles may also be used).

After centrifugation the pellet is then resuspended in a specimen diluent buffer which is adapted to the amplification process. In the preferred method the pellet obtained after centrifugation is directly resuspended in a solution which is suitable for performing RT-PCR and PCR. In the most preferred method a solution adapted for performing subsequent individual RT-PCRs for HIV, HCV and HBV as exemplified in Example 1 is used. It is noted however, that the person skilled in the art is able to adapt the method specified in Example 1 in such a way that also nucleic acids of other infectious agents can be assayed either individually or in combination. Also, the volume of lysis buffer may have to be adapted in function of the protein content of samples to be tested.

The efficiency of the assay method of the present invention is supported by the results shown in the appended figures which figures are meant to show:

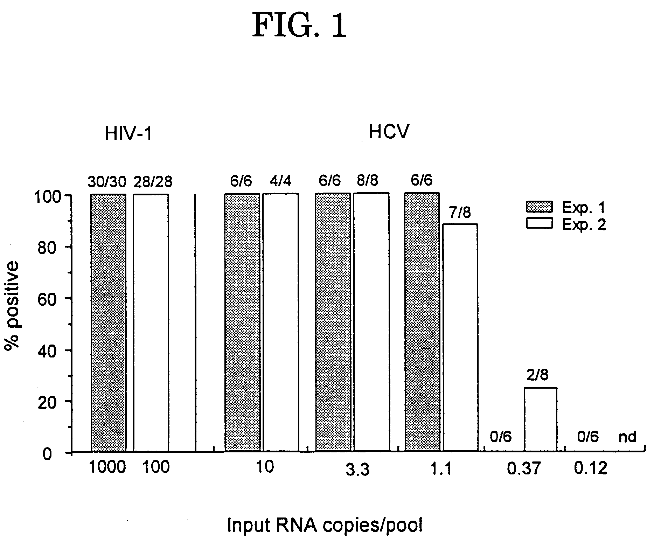

Fig 1. Detection limit of HCV RNA in pooled plasma samples containing decreasing amounts of HCV RNA copies (data from two separate experiments). Dilutions of HCV RNA positive sample were used to spike 5 ml pools (from 10 to 0.12 copies/pool). Spikes of HIV-1 RNA were also introduced in each pool tested (1 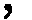 000 copies/pool for experiment no. 1 and 100 copies/ml for experiment no. 2).

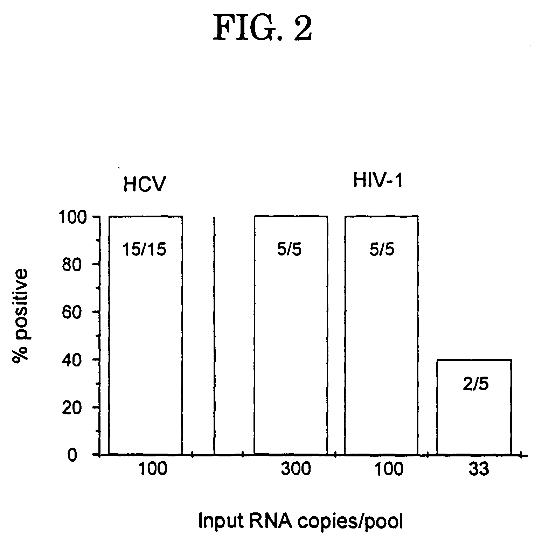

Fig 2. Detection limit of HIV-1 RNA in pooled plasma samples containing decreasing amounts of HIV RNA copies. Dilutions of HIV RNA positive sample were used to spike 5 ml pools (from 300 to 33 copies/pool). Spikes of HCV RNA were also introduced in each pool tested (100 copies/pool).

The method of the present invention is used to increase the sensitivity of diagnostic tests for the detection of nucleic acid of an infectious agent in pooled samples of liquid biological material. The term "liquid biological material" includes but is not limited to any body fluid such as urine, liquor, blood (plasma or serum) and sputum. The pooled samples are usually derived from different individuals (e.g. a human being). This is however no necessity, in that the sample used for the procedure of the present invention may also be a large volume of one single sample derived from one individual in order to increase the sensitivity of the diagnostic method. The term "nucleic acid of an infectious agent" includes any nucleic acid (e.g. DNA or RNA) of a biological organism which is capable of infecting a given individual. Examples of such biological organisms are fungi, bacteria and viruses. The method of the present invention is particularly useful for increasing the sensitivity of the detection of nucleic acids which are the cause for hepatitis (e.g. nucleic acids from hepatitis viruses such as e.g. hepatitis B virus [HBV] and hepatitis C virus [HCV]) or are the cause for an immunodeficiency disease, such as AIDS (e.g. nucleic acids from human immune deficiency viruses such as e.g. HIV-1 and HIV-2).

The method of the present invention is preferably used to detect infections by multiple infectious agents using one common purification step for purification and concentration of the infectious agents. In this way the infectious agents are concentrated and can then be detected with higher sensitivity by suitable amplification/detection methods such as e.g. RT-PCR and/or PCR. The preferred method allows the concomitant detection of HCV and HIV-1 RNA from pools of plasma samples.

The detection step in the assay method of the present invention can be designed to be directed against the nucleic acid of each infectious agent individually or may be designed to be capable of detecting the presence of the nucleic acids of several infectious agents at the same time (so-called multiplex RT-PCR and/or PCR). In the latter case the so-called reverse dot-blot procedure (see e.g. Chehab et al., in: Innis et al. eds., 1995 PCR Strategies, Academic Press, Inc., San Diego, CA., European Patent No. EP-A-237 362 and European Patent Publication No. EP-A-529 493) is preferably used, wherein the amplified material, which is preferably labeled, is added to a solid support (e.g. a membrane, a filter or the wells of a microtiter plate) which comprises sequence specific oligonucleotide (SSO) probes suitable for the detection of the desired infectious agents. In this procedure the unlabeled SSO probes are exposed to the labeled amplified nucleic acid of the pooled samples under appropriately stringent hybridization conditions. Unhybridized labeled sample nucleic acid is then removed by washing under suitably stringent conditions, and the filter is then monitored for the presence of label bound to the probe sequences. The amplified material can be labeled by either using labeled nucleic acid precursors (dNTPs) or by using labeled oligonucleotide primers.

Labeled nucleic acid precursors (dNTPs) are commercially available. Oligonucleotides for use in connection with the present invention can be prepared and can be labeled by incorporating a label detectable by spectroscopic, photochemical, biochemical, immunochemical, or chemical means based on methods well-known in the art. Useful labels include ³²P, fluorescent dyes, electron-dense reagents, enzymes (as commonly used in ELISAs), biotin (e.g. useful together with an avidin-enzyme conjugate, an enzyme substrate and a chromogen), or haptens and proteins for which antisera or monoclonal antibodies are available.

In case the detection step involves a large number of oligonucleotide probes, the said probes are preferably arranged in arrays. In such arrays a multitude of oligonucleotide probes are fixed onto the surface of so-called microchips, preferably composed of siliconized glass. In the detection step the surface of the array is then scanned for positive signals emitted by the labeled targets bound to the probes. The preferred labels are molecules which are capable of emitting a fluorescent signal under appropriate conditions. For more details on this microchip technology it is referred to publications of Kozal, M.J. et al., Nature Medicine, 1996, 2 (7):735-739; Lipshutz et al., Biotechniques, 1995, 19 (3):442-447 and Eggers M. and Ehrlich D., Hematol. Pathol., 1995, 9 (1):1-15.

The method of the present invention is particularly useful in the following fields of application:
- testing of blood donations;
- testing of intermediates and final preparations of biological origin in pharmaceutical products for human use;
- testing of products for human consumption (e.g. yogurt);
- testing of intermediates and final preparations of biological origin as pharmaceutical products for veterinarian use;
- testing of human samples with potentially low concentration of infectious agents for example following antiviral treatment or samples such as spinal fluid or synovial fluid.

The method of the present invention concentrates nucleic acids from the infectious agent(s) in a small volume and provides a purified preparation of nucleic acids which allows a more sensitive detection of such nucleic acids with a suitable detection method. The preferred method is specified in Example 1. If desired the method can be adapted by varying the concentration and the type of PEG and by adapting the concentration of the salt (e.g. NaCl). In the preferred method the PEG and salt concentrations are selected in such a way that several infectious agents can be precipitated at the same time. In the first step of the most preferred procedure PEG and the salt are added in appropriate concentration for precipitation of HIV, HCV and HBV. It was found that HIV and HBV can be precipitated even in the absence of salt and PEG. On the other hand HCV can only be precipitated in the presence of salt and not by a simple centrifugation step as described by Schockmel et al. (supra). The optimal concentrations of PEG and NaCl have been determined for the above-mentioned three infectious agents, as well as the optimal incubation time and centrifugation speed to be used for these agents (see Example 1). These various factors will vary depending on the infectious agent(s) which is/are to be precipitated. It was found that the first precipitation step allows the removal of more than 95% of the serum proteins in the sample tested.

The present invention also provides kits for determining the presence of a nucleic acid of an infectious agent in pooled samples of liquid biological material, preferably a virus nucleic acid in a blood sample (plasma or serum), wherein said kit comprises a solution of the precipitating agent as defined above and other reagents necessary for detecting the presence of the nucleic acid, such as e.g. a sequence specific oligonucleotide probe. The later probe is preferably bound to a solid support. The said kit may further comprise a solution of a mono- or divalent salt, preferably NaCl or KCl. The said kit may further comprise reagents necessary for performing an amplification reaction, such as e.g. a polymerase (e.g. a thermostable DNA polymerase such as Taq polymerase or Tth polymerase) and primer(s), preferably pairs of primers. The said primers are preferably labeled. The above-mentioned reagents are preferably packed in one or more multicontainer units, which comprises all useful components for practicing the method of the present invention and possibly an amplification of the nucleic acid of the infectious agent and its detection. The kit may also contain instructions for carrying out said methods.

Although the preferred embodiment incorporates RT-PCR amplification, amplification of target sequences in a sample may be accomplished by any known method, such as ligase chain reaction (LCR), transcription amplification, and self-sustained sequence replication, each of which provides sufficient amplification so that the target sequence can be detected by nucleic acid hybridization to an SSO probe. It may also be suitable to detect the nucleic acid concentrated by the method of the present invention with the branched DNA signal amplification technique described by Horn T. and Urdea M.S. in Nucl. Acids Res., 1989, 17 (17): 6959-6967 and Running J.A. and Urdea M. in Biotechnique, 1990, 8 (3): 276-279.

The preferred amplification reaction is the polymerase chain reaction (PCR) amplification process is well known in the art and described in U.S. Patent Nos. 4,683,195; 4,683,202; and 4,965,188. Commercial vendors, such as Perkin Elmer (Norwalk, CT), market PCR reagents and publish PCR protocols. For ease of understanding the advantages provided by the present invention, a summary of PCR is provided.

In each cycle of a PCR amplification, a double-stranded target sequence is denatured, primers are annealed to each strand of the denatured target, and the primers are extended by the action of a DNA polymerase, preferably a thermostable DNA polymerase, i.e. an enzyme that is relatively stable to heat and catalyzes the polymerization of nucleoside triphosphates to form primer extension products that are complementary to one of the nucleic acid strands of the target sequence. The DNA polymerase initiates synthesis at the 3' end of the primer and proceeds in the direction toward the 5' end of the template until synthesis terminates. Purified thermostable DNA polymerases are commercially available from Perkin-Elmer, Norwalk, CT. Further reagents present in a typical polymerase chain reaction mixture are oligonucleotide primers, nucleotide triphosphates, and a suitable buffer. Information on the preferred amplification reaction mixtures and the preferred temperature cycling conditions are known to the person skilled in the art and are provided in the package inserts in the AMPLICOR™ test kits which are commercially available from F. Hoffmann-La Roche Ltd. The amplification process is repeated typically at least 25 times. The two primers anneal to opposite ends of the target nucleic acid sequence and in orientations such that the extension product of each primer is a complementary copy of the target sequence and, when separated from its complement, can hybridize to the other primer. Each cycle, if it were 100% efficient, would result in a doubling of the number of target sequences present.

Either DNA or RNA target sequences can be amplified by PCR. In the case of an RNA target, such as in the amplification of e.g. HCV genomic nucleic acid, the first step consists of the synthesis of a DNA copy (cDNA) of the target sequence. The reverse transcription can be carried out as a separate step, or, preferably, in a combined reverse transcription-polymerase chain reaction (RT-PCR), a modification of the polymerase chain reaction for amplifying RNA. The RT-PCR amplification of RNA is well known in the art and described in U.S. Patent Nos. 5,322,770 and 5,310,652; Myers and Gelfand, 1991, Biochemistry 30(31):7661-7666; U.S. Patent No. 5,527,669; Young et al., 1993, J. Clin. Microbiol. 31(4):882-886; and Young et al., 1995, J. Clin. Microbiol. 33(3):654-657.

Due to the enormous amplification possible with the PCR process, low levels of DNA contamination from samples with high DNA levels, positive control templates, or from previous amplifications can result in PCR product, even in the absence of purposefully added template DNA. Laboratory equipment and techniques which will minimize cross contamination are discussed in Kwok and Higuchi, 1989, Nature, 339:237-238 and Kwok and Orrego, in: Innis et al. eds., 1990 PCR Protocols: A Guide to Methods and Applications, Academic Press, Inc., San Diego, CA. Enzymatic methods to reduce the problem of contamination of a PCR by the amplified nucleic acid from previous reactions are described in U.S. Patent Nos. 5,418,149 and 5,035,996, and in Young et al., 1995, supra.

Amplification reaction mixtures are typically assembled at room temperature, well below the temperature needed to insure primer hybridization specificity. Non-specific amplification may result because at room temperature the primers may bind non-specifically to other, only partially complementary nucleic acid sequences, and initiate the synthesis of undesired nucleic acid sequences. These newly synthesized, undesired sequences can compete with the desired target sequence during the amplification reaction and can significantly decrease the amplification efficiency of the desired sequence. Non-specific amplification can be reduced using a "hot-start" wherein primer extension is prevented until the temperature is raised sufficiently to provide the necessary hybridization specificity.

In one hot-start method, one or more reagents are withheld from the reaction mixture until the temperature is raised sufficiently to provide the necessary hybridization specificity. Hot-start methods which use a heat labile material, such as wax, to separate or sequester reaction components are described in U.S. Patent No. 5,411,876 and Chou et al., 1992, Nucl. Acids Res. 20(7):1717-1723. In another hot-start method, a reversibly inactivated DNA polymerase is used which does not catalyze primer extension until activated by a high temperature incubation prior to, or as the first step of, the amplification. An example of such a reversibly inactivated DNA polymerase is AmpliTaq™GOLD (European Patent Application No. EP-A-771 870; for a review see also Birch et al., 1996, Nature 381:445-446). Non-specific amplification also can be reduced by enzymatically degrading extension products formed prior to the initial high-temperature step of the amplification, as described in U.S. Patent No. 5,418,149.

The PCR method leads to an increase in the amount of nucleic acid of the infectious agent to a detectable level. Methods for detecting amplified nucleic acids are well known in the art. For example, the presence and quantity of amplified product can be assayed directly using gel electrophoresis using protocols well known in the art (see, for example, Sambrook et al., 1989, Molecular Cloning - A Laboratory Manual, Cold Spring Harbor Laboratory, Cold Spring Harbor, New York).

In connection with the method of the present invention the detection of the amplified product is preferably carried out by using oligonucleotide probes. Preferably sequence-specific oligonucleotide (SSO) probes are used, i.e. one or more oligonucleotide probes wherein each probe has a sequence, called a 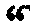 hybridizing region 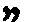 , which is exactly complementary to a sequence to be detected. Typically such a hybridizing region will under 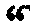 sequence-specific, stringent hybridization conditions 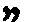 hybridize only to the exact complementary target sequence. Relaxing the stringency of the hybridizing conditions will allow sequence mismatches to be tolerated; the degree of mismatch tolerated can be controlled by suitable adjustment of the hybridization conditions. Depending on the sequences being analyzed, one or more sequence-specific oligonucleotides may be employed.

A sequence specific to a particular infectious agent, e.g. a particular isolate thereof, is a sequence unique to the said infectious agent or isolate thereof, i.e. a sequence that is not shared by other infectious agents or particular isolate of such infectious agent. A probe containing a subsequence complementary to a sequence specific to an infectious agent will typically not hybridize to the corresponding portion of the genome of another infectious agent under stringent conditions (e.g. washing the solid support in 2 x SSC, 0.1% SDS at 70°C). Suitable protocols for detecting hybrids formed between probes and target nucleic acid sequences are known in the art. An example of such a protocol is described in U.S. Patent No. 5,527,669; Young et al., 1993, supra; and Young et al., 1995, supra.

Another detection method used with PCR is the so-called 5'-nuclease assay method which is described in U.S. Patent No. 5,210,015 and by Holland et al. in Proc. Natl. Acad. Sci. USA, 1991, 88:7276-7280. In the 5'-nuclease assay, labeled detection probes are involved in the PCR amplification reaction mixture. The probes are modified so as to prevent the probes from acting as primers for DNA synthesis. Any probe which is hybridized to target DNA during a synthesis step, i.e., during primer extension, is degraded by the 5'-nuclease activity of the DNA polymerase (e.g. rTth DNA polymerase). The presence of degraded probe indicates both that hybridization between probe and target DNA occurred and that amplification occurred. Methods for detecting probe degradation are described in U.S. Patent No. 5,210,015, in EP-A-699 768 and in EP-A-713 921.

A further method for detecting the presence of a given amplified nucleic acid is described in Higuchi et al., 1992, Bio/Technology 10:413-417; Higuchi et al., 1993, Bio/Technology 11:1026-1030; and European Patent Publication Nos. EP-A-487,218 and EP-A-512,334. In this method the increase in the total amount of double-stranded DNA in the reaction mixture is monitored. The detection of double-stranded target DNA relies on the increased fluorescence that ethidium bromide (EtBr) and other DNA binding labels exhibit when bound to double-stranded DNA. Amplification increases the amount of double-stranded DNA and results in a detectable increase in fluorescence. Because non-specific amplification and primer-dimer formation also results in the formation of double-stranded DNA, suitable primers which lead to a low amount of non-specific amplification and primer-dimer formation should be used in this method.

The combination of the method of the present invention with an amplification method is however not necessarily limited to the use in a detection assay, because the amplified nucleic acid can also be used in cloning or sequencing methods (see e.g. U.S. Patent No. 4,683,195).

Further information on conventional techniques of molecular biology and nucleic acid chemistry, which are within the skill of the art can be found e.g. in Sambrook et al., 1989, Molecular Cloning - A Laboratory Manual, Cold Spring Harbor Laboratory, Cold Spring Harbor, New York; Oligonucleotide Synthesis (M.J. Gait, ed., 1984); Nucleic Acid Hybridization (B.D. Hames and S.J. Higgins. eds., 1984); and a series, Methods in Enzymology (Academic Press, Inc.). All patents, patent applications, and publications mentioned herein, both supra and infra, are incorporated herein by reference.

Evidence is presented in the examples below that a PCR-based analysis of pools of plasma donors can be applied for the concomitant detection of HCV and HIV-1 RNA using an unique nucleic acid preparation. This was achieved by including precipitation of viral particles which allows an increase of the input of purified viral RNA in the PCR reactions. The RNA input in the PCR reaction for each sample of the pool corresponds to a theoretical plasma volume 2 to 10-fold higher using this pooling procedure than that used for individual testing by commercial assays. The procedure of the present invention allows the detection of less than 3.3 HCV RNA copies per pool of 50 blood donations, corresponding to the detection of an individual positive plasma sample containing less than 33 copies/ml. This corresponds to a detection limit lower than that of the most sensitive commercial assay available today (Cobas AMPLICOR™ HCV). The limit of HIV-1 RNA detection is therefore around 100 HIV-1 RNA copies per pool of 50 blood donations.

In the course of the investigations which led to the present invention the focus was on HCV RNA detection since the recent development of boosted assays for HIV-1 RNA detection have shown that a simple pre-purification step of viral particles using a high speed centrifugation can increase the sensitivity of commercial assay by a factor of 10 to 30 (Schockmel G et al., J. Acquir. Immun. Defic. Syn., 1997, 14:179). High speed centrifugation of HCV virions did not result in an increase in viral particles recovery in the pellet. The possibility to concentrate HCV virions through polyethylene glycol (PEG) precipitation was first explored. This was partially successful since around 50% of the total virus was recovered in the pellet using 3% PEG, 145 mM NaCl and low speed centrifugation. The use of higher concentration of PEG increased the recovery of the virus in the pellet, but the resuspension of the pellet in the lysis reagent was very difficult. Moreover an inhibition of the PCR reactions was observed at high frequency. The use of a large volume of lysis reagent and the total removal of ethanol were important factors to preclude inhibition of the PCR reactions. Using the PEG precipitation assay, no inhibition of the PCR reactions was observed in more than 20 pools of 50 blood donations tested for both HIV and HCV RNA. These results indicate that residual PEG does not inhibit PCR reactions and that plasma with inhibitory activity are either infrequent in the population of blood donors or that PEG precipitation remove inhibitory activities by discarding plasma components (Schockmel et al., J. Acquir. Immun. Defic. Syn., 1997, 14:179). Moreover, the testing of pooled samples does not increase the background level. A clear cut-off between positive and negative results was always observed.

The main interest of PCR-based screening assays resides in the identification of plasma samples with viral nucleic acids in the absence of specific antibodies. These samples correspond mainly to pre-seroconversion samples from individuals with acute infection (De Saussure et al., Transfusion, 1993, 33:164; Busch MP. , Vox Sang, 1994, 67 (suppl 3):13; Busch MP and Satten GA, Am. J. Med., 1997, 102:117). Pre-seroconversion samples from eight individuals were found to be HCV RNA positive without detectable anti-HCV antibodies. When these samples are introduced in the pools, the PEG assay of the present invention detected all of them, including 3 plasma samples with less than 5 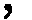 000 HCV RNA copies/ml. This indicates that binding of specific antibodies to HCV virion is not a prerequisite for PEG precipitation of viral particles. Unexpected low concentrations of HCV RNA were observed in some of the HCV pre-seroconversion samples. The results shown in the examples indicate that very sensitive assays are required for the detection of HCV seroconvertors by PCR-based pooling assays. The issue of HIV-1 RNA detection is different since high levels of HIV-1 RNA have been systematically reported in pre-seroconversion samples (Busch MP and Satten GA, Am. J. Med., 1997, 102:117; Kinloch-de Loes et al., N. Engl. J. Med., 1995, 333: 408; Baumberger et al., AIDS, 1993, 7(suppl. 2):59). It should be noted that the relationship between minimal detectable levels of viral nucleic acid in blood and the minimal infectious dose is a critical issue.

Another issue of concern is the viral subtype diversity. In this context, the genomic regions targeted by PCR-based assays must be highly conserved across divergent viral strains. The selection of primers located in the highly conserved 5 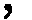 untranslated region of the HCV genome allows the detection of the great majority of HCV subtypes (Buck et al., Proc. Natl. Acad. Sci. USA, 1992, 89:187; Young et al., J. Clin. Microbiol., 1993, 31:882) as shown below in the examples. Detection of HIV subtypes is more problematic since high variability was reported in the gag gene from which primers for HIV-1 amplification are derived (Myers et al., Theoretical Biology and Biophysics, Los Alamos National Laboratory, Los Alamos, NM, USA 1995). Moreover, recombinant HIV virus strains with genetic regions derived from multiple clades have recently been observed (Diaz et al., 1995, J. Virol. 69:3273). Recently, the implementation of new primers which allows the detection of the majority of HIV-1 subtypes in the AMPLICOR™ HIV Monitor Test has reduced the risk of missing particular HIV-1 subtypes (Michael et al., IV Conference on Retroviruses and Opportunistic Infections. Washington, DC, (abstr. 279), 1997). However, particular attention should be given to the constant and worldwide assessment of HIV-1 genetic evolution.

The examples of the present invention presented below are provided only for illustrative purposes and not intended to limit the scope of the invention. Numerous embodiments of the invention within the scope of the claims that follow the guidance given in the examples will be apparent to those of ordinary skill in the art from reading the foregoing text and the following examples.

### Example 1

In this example a preferred protocol for the detection of HIV, HBV and HCV in pooled plasma is described. It should be understood that variations of this protocol, as long as these variations are within the limits specified by the appended claims, are possible. Such variations are within the scope of the claimed invention.

### Protocol

1. Add 5000 µl plasma pool with or without spike RNA, 270 µl 3M NaCl and 320 µl 50% PEG 6000 (e.g. Fluka 81304) in H₂O into a 15 ml sterile tube (e.g. a conical propylene tube such as Falcon Tube # 2097; available from Becton Dickinson Co.);
2. mix, incubate on ice for 30 minutes;
3. centrifuge at 5 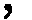 000 rpm for 30 minutes at 4°C (e.g. Heraeus minifuge GL);
4. discard supernatant (e.g. with a disposable Pasteur pipette);
5. resuspend in 2.5 ml of a lysis buffer (60 mM Tris/HCl pH 7.5, 68 % w/v guanidine thiocyanate, 3 % w/v dithiothreitol);
6. incubate 10 minutes at 37°C (e.g. in a standard incubator or in a waterbath);
7. add 2.6 ml isopropanol (another alcohol such as e.g. methanol, ethanol, propanol and liquid forms [at room temperature] of butanoles, pentanoles and hexanoles may also be used);
8. incubate 2 minutes at room temperature (about 15 to 25°C);
9. centrifuge at 5 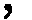 000 rpm for 15 minutes at room temperature (e.g. Heraeus minifuge GL);
10. discard supernatant;
11. add 1 ml of 70% v/v ethanol in water (instead of ethanol another alcohol such as e.g. methanol, propanol, isopropanol and liquid forms [at room temperature] of butanoles, pentanoles and hexanoles may also be used);
12. transfer into a 2 ml tube (e.g. a sterile tube with a cap such as Sarstedt, type No. 72.693.105);
13. centrifuge at 15 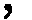 000 rpm for 5 minutes at room temperature (e.g. in an Eppendorf 5415 centrifuge);
14. discard supernatant;
15. add 55 µl HIV specimen diluent [10 mM Tris-HCl, 0.1 mM EDTA, 20 µg/ml poly rA RNA, 0.05 % w/v sodium azide] and resuspend pellet;
16. take 25 µl of resuspended pellet and add 25 µl HIV specimen diluent;
17. use 50 µl of this mixture for amplification and detection for HIV;
18. take 25 µl of resuspended pellet from step 15 and add 25 µl of 2x HCV specimen diluent (100 mM Bicine, 200 mM potassium acetate, 14 mM manganese (II) acetate, 0.05 % w/v sodium azide);
19. use 50 µl of this mixture for amplification and detection of HCV.

The above procedure when combined with the standard procedures provided with the AMPLICOR™ test kits for HCV, HIV and HBV allows the specific detection of HCV, HIV and HBV in pooled samples.

With respect to step 1 it is noted that the sample input volume is important in the sense that only by precipitation one can get rid of agents which may potentially inhibit amplification. Addition of salt in this preferred concentration is critical for precipitation of HCV. It was found that the recovery of HCV nucleic acid was less than 50 % in absence of PEG and salt after centrifugation at 25'000 x g for 1 hour. A different type of PEG in a different concentration may also be used. It should however be noted that when the molecular weight of the PEG and/ or its concentration are changed, it may become necessary to also change the type and/or the concentration of the salt which is added to aid the precipitation. In case the viruses to be precipitated from the pooled samples differ from those specified above, then it may also become necessary to optimize the concentrations of PEG and salt with respect to these viruses.

With respect to step 5 it is noted that the resuspension in a large volume of lysis buffer is very critical because this helps to get rid of the precipitating agent (PEG). If the volume is lower than 1 milliliter, partial or complete inhibition of the PCR reaction can occur.

The condition specified in step 15 are particularly suitable for detecting HCV. In case additionally a test for HCV is performed the latter solution can be added as a two-fold concentrated solution. If only HCV shall be tested, the pellet can be resuspended directly in HCV specimen diluent [50 mM Bicine, 100 mM potassium acetate, 7 mM manganese (II) acetate, 0.05 % w/v sodium azide). This volume is much lower compared to the input sample volume (55 µl / 5 ml serum) than in the standard AMPLICOR™ Monitor procedure (1 ml / 100 µl serum). The consequence is that the equivalent of 5 ml starting volume is used to initiate the PCR instead of 5 µl. This leads to a 1000-fold increase in sensitivity when one sample rather than a pool of a number of samples is used. Of course when a number of samples are pooled the sensitivity relative to the individual sample decreases accordingly.

It was found that the method according to Example 1 allows to detect reproducibly about 100 copies of HCV nucleic acid in 5 ml of pooled plasma. The recovery of the HCV nucleic acid was calculated to be between 70 to 100 % (based on a method, wherein a starting plasma containing 8000 copies of HCV nucleic acid per milliliter was first tested with a standard HCV AMPLICOR™ test and then retested after a 400 - fold dilution of the sample).

The assay method of the present invention is particularly useful in assay methods for detecting HCV. It was observed that high speed centrifugation (50 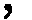 000 g for 60 minutes) does not lead to a concentration of HCV viral particles in the pellet. A polyethylene glycol (PEG), NaCl precipitation procedure was therefore performed as an initial step before lysis of viral particles. The optimal concentration of PEG and NaCl required to achieve maximal recovery and ease of pellet resuspension was evaluated. It was found that the addition of 3 % PEG and 145 mM NaCl to plasma pools was optimal. Potential inhibition of the PCR reactions was evaluated using the quantitative detection assay (AMPLICOR™ HCV Monitor) on 15 pools containing 100 µl of 50 unselected blood donations.

**Table 1**

| Detection of HCV RNA in pooled plasma samples | | | | | | | | | | |
|---|---|---|---|---|---|---|---|---|---|---|
| Pools | Pool (5 ml)* | | | | Pool (5 ml) + spike HCV | | | | | |
| | HCV (OD) | DF | IQS (OD) | DF | Spike HCV (copies) | HCV (OD) | DF | IQS (OD) | DF | HCV copies |
| P1 | 0.079 | 1 | 0.454 | 5 | 5000 | 0.521 | 25 | 0.303 | 5 | 2879 |
| P2 | 0.095 | 1 | 0.303 | 5 | 5000 | 0.204 | 25 | 0.226 | 5 | 1278 |
| P3 | 0.077 | 1 | 0.676 | 25 | 200 | 0.301 | 25 | 0.719 | 25 | 93 |
| P4 | 0.082 | 1 | 0.551 | 25 | 200 | 0.387 | 25 | 0.538 | 25 | 178 |
| P5 | 0.081 | 1 | 0.493 | 25 | 200 | 0.647 | 5 | 0.406 | 25 | 90 |
| P6 | 0.079 | 1 | 0.409 | 25 | 200 | 0.371 | 25 | 0.543 | 25 | 167 |
| P7 | 0.083 | 1 | 0.539 | 25 | 200 | 0.489 | 5 | 0.403 | 25 | 66 |
| P8 | 0.079 | 1 | 0.355 | 25 | 85 | 0.441 | 5 | 0.448 | 25 | 52 |
| P9 | 0.081 | 1 | 0.438 | 25 | 85 | 0.309 | 5 | 0.381 | 25 | 40 |
| P10 | 0.079 | 1 | 0.436 | 25 | 85 | 0.519 | 5 | 0.417 | 25 | 68 |
| P11 | 0.082 | 1 | 0.44 | 25 | 85 | 0.431 | 5 | 0.457 | 25 | 49 |
| P12 | 0.078 | 1 | 0.369 | 25 | 85 | 0.675 | 5 | 0.386 | 25 | 101 |
| P13 | 0.080 | 1 | 0.358 | 5 | 45 | 0.661 | 1 | 0.243 | 25 | 29 |
| P14 | 0.089 | 1 | 0.364 | 5 | 45 | 0.559 | 1 | 0.336 | 25 | 15 |
| P15 | 0.082 | 1 | 0.347 | 5 | 45 | 0.706 | 1 | 0.281 | 25 | 25 |

| | | | | | | | | | | |
|---|---|---|---|---|---|---|---|---|---|---|
| * Each pool contains 100 µl of plasma from 50 blood donors | | | | | | | | | | |

Table 1 shows results of the 15 pools tested with and without the addition of a plasma containing known HCV RNA copy numbers. The following abbreviations are used in this table: OD means optical density values (AMPLICOR™ HIV Monitor), IQS means internal quantitative standard and DF means dilution factor of the detection procedure. All the 15 pools tested were negative for HCV RNA (OD HCV < 0.2) and all pools containing known numbers of HCV RNA copies (from 5 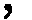 000 to 45 copies/5 ml pool) were found positive. No inhibition of PCR reactions was observed, as shown by OD values of the internal HCV quantitative standard (IQS), among the 30 PCR reactions performed including plasma samples of 750 blood donors.

The limit of sensitivity of the PEG method for HCV RNA detection in 5 ml pools was evaluated using the qualitative detection assay (Cobas AMPLICOR™ HCV test). The sensitivity of this assay is around 100 HCV RNA copies/ml, which is at least 10 times lower than the quantitative assay. Figure 1 shows the percentage of HCV RNA positive pools according to the input of HCV RNA copies per pool. The HCV RNA positive plasma used in the two experiments was quantitated using 4 dilutions in duplicate, the mean HCV RNA level was 23 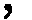 330 copies/ml (range, 16 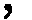 340 to 30 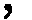 210). This patient was infected with HCV genotype 4h. All replicates (10/10 and 14/14) of 5 ml pools containing respectively 10 and 3.3 of HCV RNA copies, 13/14 replicates containing 1.1 copies and 2/14 replicates containing 0.37 copy were found positive, whereas none of the replicates with 0.12 copy were positive. No inhibition of PCR reactions was observed, since the OD values of internal control (IC) were always above 3.0 in the 58 pools tested. Spikes of HIV-1 RNA were also introduced in each pool tested, 1 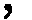 000 copies per pool for the first experiment and 100 copies per pool for the second. All the 58 pools tested were found positive. The input of 100 HIV-1 RNA copies were always clearly detected with an OD HIV ranging from 2.056 at 1/1 dilution factor (DF) to 0.219 (1/125 DF) and no inhibition of PCR reactions was observed (OD IQS > 3.5).

The limit of sensitivity of the PEG method for HIV-1 RNA detection in 5 ml pools was evaluated using dilutions of HIV-1 RNA positive plasma (Figure 2). This sample was quantitated at 2 dilutions in duplicate, the mean HIV RNA level was 40 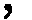 000 copies/ml (range, 35 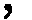 800 to 49 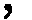 450). All the 5 replicates of 5 ml pools containing 300 and 100 HIV RNA copies and 2/5 replicates containing 33 HIV-1 RNA copies were found positive. Spikes of HCV RNA (100 copies/pool) were also introduced in each pool, and all the 15 pools tested were found positive. No inhibition of PCR reactions was observed (OD HIV-1 IQS > 3.0 and OD HCV IC > 3.7).

The partition of HCV RNA between the pellet and the supernatant after PEG precipitation was evaluated using the quantitative detection assay (Table 2). Four different plasma samples with known HCV RNA copy numbers from one individual with established HCV infection (K) and three pre-seroconversion individuals (S1, S2, S4) were used to spike 5 ml pools containing 100 µl of plasma of 50 blood donations. The concentration of HCV RNA in the supernatant after PEG precipitation was evaluated using 100 µl of the supernatant with the standard procedure (AMPLICOR™ HCV Monitor). The total recovery (pellet + supernatant) of HCV RNA varied from 53 to 132% and recovery of HCV RNA in the pellet varied from 40 to 68%. Similar HCV RNA recovery was observed for established HCV infection and pre-seroconversion samples. The genotype of the HCV strain used in sample K is 1a. The HCV genotypes of samples S1, S2, S4 are reported in Table 3. Sample S1 was used at 1/10 dilution in this experiment.

**Table 2**

| Evaluation of the recovery of HCV RNA copies after PEG precipitation | | | | | |
|---|---|---|---|---|---|
| Spike HCV (copies/100 µl) | | Pellet (copies/pellet) | Supernatant (copies/4.9 ml) | Total recovery (%) | Pellet recovery* (%) |
| S1 | 13 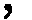 558 | 9 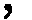 871 | 6 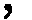 904 | 124 | 59 |
| S1 | 16 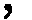 924 | 7 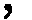 849 | 9 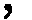 692 | 104 | 45 |
| S2 | 3 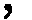 124 | 1 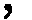 854 | 2 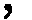 264 | 132 | 45 |
| S2 | 3 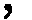 281 | 1 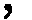 891 | 882 | 85 | 68 |
| S4 | 471 | 250 | neg | 53 | 53 |
| S4 | 466 | 288 | neg | 62 | 62 |
| K1 | 23 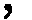 209 | 6 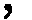 978 | 8 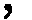 688 | 68 | 45 |
| K1 | 21 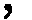 919 | 6 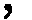 779 | 11 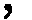 221 | 82 | 66 |
| K2 | 4 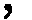 999 | 2 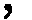 383 | 3 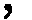 528 | 118 | 40 |
| K2 | 5 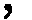 670 | 2 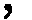 354 | 2 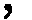 867 | 92 | 45 |

| | | | | | |
|---|---|---|---|---|---|
| * calculated from HCV RNA copies recovered from pellet and supernatant | | | | | |

In order to see whether the presence of complexing antibodies had an influence on the capacity to precipitate the viruses, the detection of HCV RNA was analyzed in 5 ml pools using pre-seroconversion samples (HCV RNA positive, anti-HCV antibody negative) from 8 patients. Ten fold dilutions of the 8 pre-seroconversion samples were used to spike 5 ml pools.

**Table 3**

| Analysis of pre-seroconversion HCV samples | | | | | | |
|---|---|---|---|---|---|---|
| Spike HCV samples | | | Pool + 100 µl spike HCV at dilutions: | | | |
| | copies/ml | genotype | 1/1 | 1/10 | 1/100 | 1/1 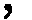 000 |
| S5 | 1 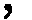 250 | 3c | 2/2 | 1/2 | 0/2 | nd |
| S3 | 4 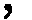 300 | 4h | 2/2 | 1/2 | 0/2 | nd |
| S4 | 4 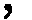 700 | 2a/c | 2/2 | 1/2 | 0/2 | nd |
| S2 | 32 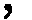 000 | 3a | nd | 2/2 | 2/2 | 0/2 |
| S8 | 110 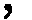 000 | 1b | nd | 2/2 | 2/2 | 2/2 |
| S6 | 200 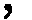 000 | 3a | nd | 2/2 | 2/2 | 1/2 |
| S7 | 253 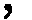 000 | 1a | nd | 2/2 | 2/2 | 2/2 |
| S1 | 762 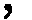 000 | 4c/d | nd | nd | 2/2 | 2/2 |
| nd: means not done | | | | | | |

Table 3 reports the number of positive pools according to dilutions of the pre-seroconversion samples. The HCV RNA level of the pre-seroconversion samples was determined in duplicate and varied from 1 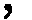 250 to 762 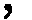 000 copies/ml. Using 100 µl of undiluted plasma samples, the 3 pre-seroconversion samples with the lowest HCV RNA levels (S3, S4, S5) were found positive in duplicate and 1/2 replicates were positive at dilution 1/10 (input 10 µl/pool). All the other pre-seroconversion samples were constantly detected in duplicate at dilution 1/100. No difference in HCV RNA detection limit was observed among the different HCV genotypes tested (1a, 2a/c, 3a, 3c, 4c/d, 4h) on the basis of dilutions analysis.

### Example 2

### Pools of blood donations

Whole blood was collected in 7 ml EDTA tube from volunteer blood donors (Transfusion Center, Geneva University Hospital) and plasma was separated by centrifugation at 1 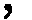 000 g for 10 minutes at room temperature. Pools of 50 blood donations were assembled using 2 ml of each individual plasma, the pools were centrifuged again at 1 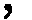 000 g for 10 minutes. Aliquots of 5 ml pools and individual plasma were stored at -75°C within 4 hours. The study protocol was approved by the local Ethical Committee. Dilutions of HCV or HIV-1 positive plasma with known RNA copy numbers were added to the pools to assess the sensitivity of the PCR-based pooling assay.

### Seroconverting patients

Plasma samples from consecutive patients anti-HCV antibody negative (Axsym 3.0, Abbott Laboratories, Abbott Park, IL) and HCV RNA positive (AMPLICOR™ HCV test) were selected from our -75°C plasma collection.

### Specimen preparation

Extraction of HCV and HIV-1 RNA was performed according to the manufacturer's instructions (AMPLICOR™ Monitor) with some modifications including first, a polyethylene glycol (PEG) precipitation step prior to lysis of virus particles, and second changes in reagent volumes in order to increase the input of extracted RNA in the PCR reaction. Briefly, 145 mM NaCl and 3% PEG (PEG 6000 solution, Fluka, Buchs, Switzerland) were added to 5 ml pools in 15 ml tubes (Falcon, Becton Dickinson, Lincoln Park, NJ). The tubes were incubated on ice for 30 minutes, then centrifuged at 1 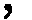 500 g for 30 minutes at 4°C. The supernatant was removed and the pellet was resuspended in 2.5 ml of lysis reagent (Cobas AMPLICOR™ HCV) and kept at room temperature for 10 minutes. After addition of 2.6 ml of isopropanol, the tube was centrifuged at 1 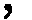 500 g for 15 minutes at room temperature. The pellet was resuspended in 1 ml of 70% ethanol, transferred in a 1.5 ml tube (Sarstedt AG, Sevelen, Switzerland) and centrifuged at 12 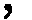 500 rpm for 5 minutes at room temperature. Ethanol was completely removed using disposable transfer pipettes and tubes were left open for 15 minutes to remove ethanol trace. The pellet was resuspended in 55 µl of HIV specimen diluent (AMPLICOR™ HIV Monitor).

### Amplification and detection of HCV and HIV-1 RNA.

For HCV PCR reactions, 25 µl of extracted sample was mixed with 25 µl of HCV specimen diluent (2X) containing 2X of internal control (IC), and 50 µl of HCV master mix. Amplification cycles and detection of HCV RNA was performed according to manufacturer's instruction on the Cobas instrument which provides complete automation for the PCR and detection steps. For HIV-1 PCR reaction, 25 µl of extracted sample was mixed with 25 µl of HIV specimen diluent and 50 µl of HIV master mix. PCR was performed using AMPLICOR™ HIV Monitor according to the manufacturer's instructions except for the number of amplification cycles which was increased from 36 to 40. The internal HIV quantitation standard (IQS) was added to the lysis reagent (6.6 µl IQS per sample), this allows to control RNA recovery during purification step.
Samples were considered positive when optical density (OD) of HCV detection was above 0.2 with an OD for the IC above 0.2 and when OD of HIV detection was above 0.2 with an OD for the IQS above 0.3.
Quantitation of HCV and HIV-1 RNA levels for individual samples was performed using AMPLICOR™ Monitor (Roche) according to the manufacturer 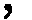 s instructions.

### HCV genotyping

HCV genotypes and subtypes were assessed using a commercial assay (INNO-LiPA HCV II, Innogenetics N.V., Zwijndrecht, Belgium) after amplification with the AMPLICOR™ HCV system.

## Claims

1. A method for increasing the sensitivity of a diagnostic test for the detection of a nucleic acid of an infectious agent in pooled samples of liquid biological material, characterized in that the nucleic acid is selectively precipitated by adding a precipitating agent, whereby the preferred precipitating agent is polyethylene glycol in a molecular weight range of 1'000 to 10'000 at a final concentration of 1 to 5 % (w/v).

2. The method of claim 1 characterized in that the precipitating agent is polyethylene glycol in a molecular weight range of 7'000 to 9'000 at a final concentration of 2.0 to 4.0 % (w/v), preferably PEG 6000 at a final concentration of about 2.8 to 2.9 % (w/v) in the pooled samples.

3. The method of claim 1 characterized in that a mono- or divalent salt, preferably a monovalent salt is added to aid in the precipitation of the nucleic acid.

4. The method of claim 3 wherein the monovalent salt is NaCl or KCl, preferably wherein the salt is added to yield a final concentration of 200 to 450 mM, most preferably 270 to 325 mM salt.

5. The method of any one of claims 1 to 4 wherein the precipitation is effected at a temperature below 5°C.

6. The method of any one of claims 1 to 5 which involves the simultaneous precipitation of multiple types of virus.

7. The method of any one of claims 1 to 6 which is followed by a lysis of the infectious agents, preferably with a chaotropic agent.

8. The method of claim 7 which is followed by an additional purification step, which is preferably a precipitation step using isopropanol or another C₁ to C₆ alcohol.

9. Use of a method as claimed in any one of claims 1 to 8 in a diagnostic test in order to increase the sensitivity for the detection of a nucleic acid of an infectious agent in pooled samples of liquid biological material or in order to increase the sensitivity of detection of individual agents in a variety of biological samples.

10. A kit for determining the presence of a nucleic acid of an infectious agent in pooled samples of liquid biological material, preferably a virus nucleic acid in a blood sample (plasma or serum), wherein said kit comprises a solution of polyethylene glycol as defined in claim 1 or 2 and preferably other reagents necessary for detecting the presence of the nucleic acid, such as e.g. a sequence specific oligonucleotide probe.

11. A kit according to claim 10 further comprising a solution of a mono-or divalent salt, preferably NaCl or KCl.

12. A kit according to claim 10 or 11 further comprising a solution of guanidine thiocyanate in a suitable buffer.

13. A kit according to any one of claims 10 to 12, further comprising reagents necessary for performing an amplification reaction and preferably a detection step.
